**Europäisches Patentamt**

**European Patent Office**

**Office européen des brevets**

(11) Publication number: **0 355 453**
**A2**

## EUROPEAN PATENT APPLICATION

(21) Application number: **89113786.1**

(22) Date of filing: **26.07.89**

(51) Int. Cl.4: **A61K 31/19**

The title of the invention has been amended (Guidelines for Examination in the EPO, A-III, 7.3).

(30) Priority: **26.07.88 JP 185725/88**

(43) Date of publication of application:
**28.02.90 Bulletin 90/09**

(84) Designated Contracting States:
**BE CH DE ES FR GB GR IT LI**

(71) Applicant: **KANEGAFUCHI KAGAKU KOGYO KABUSHIKI KAISHA**
**2-4 Nakanoshima 3-chome**
**Kita-ku Osaka-shi Osaka-fu(JP)**

(72) Inventor: **Hiraide, Atsushi**
**202, 2-37, Fukushima 1-chome Fukushima-ku**
**Osaka-shi(JP)**
Inventor: **Katayama, Masami**
**5-28, Yasui-cho**
**Nishinomiya-shi Hyogo-ken(JP)**

(74) Representative: **Vossius & Partner**
**Siebertstrasse 4 P.O. Box 86 07 67**
**D-8000 München 86(DE)**

(54) Use of 3-hydroxybutyric acid as an energy source.

(57) The present invention provides a substitution fluid preparation for the supply of energy to patients in increased protein catabolism in the living body which contains at least one of 3-hydroxybutyric acid and its salts. The substitution fluid preparation of the present invention can supply energy to patients in the state of increased protein catabolism in the living body, such as invasion-suffered patients, patients insufficient in hepatic functions, patients unable to take foods orally or patients in the state of malnutrition.

EP 0 355 453 A2

# SUBSTITUTION FLUID PREPARATION COMPRISING 3-HYDROXYBUTYRIC ACID (BETA-HYDROXYBUTYRIC ACID) AND ITS SALTS

The present invention relates to a substitution fluid preparation, and more particularly to a substitution fluid composition which is applied for the supply of energy source and for the suppression of increased protein catabolism in the living body to (1) traumatized patients or severely burned patients in the acute phase, post-operative patients, severely infectious patients and invasion-suffered patients, (2) patients in insufficient in hepatic functions and in ketone-synthesizing ability, (3) patients unable to take foods orally, patients in the state of malnutrition (especially protein-calorie malnutrition).

The substitution fluid preparation of the present invention is also useful as an alkalization agent to metabolic acidosis which is often observed in the state as aforesaid.

3-hydroxybutyric acid as the principal ingredient of the substitution fluid composition of the present invention was first found in the urine of some diabetic patients in the form of (R)-3-hydroxybutyric acid and was then deemed to be a useless metabolite occurring in the living body under morbid conditions. Also, as fat-derived energy substrate the object of concern were long-chain fatty acids and the physiological significance of this substance had been rather neglected for a long time. With regard to its usefulness, it has been found as disclosed by

YP-A-201746/83 that its (D)-form exhibits a cardiomuscular metabolism protective action and since then, uses as pharmaceutical compositions to patients of cardiomuscular metabolism in the form of L-lysine or L-arginine salt have been developed. On the other hand, as regards 3-hydroxybutyric acid, the true physiological significance of this substance has come to be realized recently as a complementary substrate as substitute of glucose and also as a retainer of caloric homeostasis in the living body.

As to the significance of this substance as energy substrate, it is already known that it is a fat-derived energy substrate, that it is hydrophilic and has an extremely good migration-to-tissues behavior and that it has been used in preference to long-chain fatty acids or glucose in vitro experiments involving tissues of various kinds [(1) Forsey R.G.P., Reid. K. Brosnan J. T. Can. J., "Physiological Pharmacology," 65, 401 - 406, 1987; (2) Robinson A. M., Wiliamson D. H., "Physiological Reviews," 60 (1), 143-187, 1980], and judging from all these, it can be safely said that this substance is an excellent energy substrate.

When the supply of glucose is infeasible in the living body, it is already known that this substance provides a useful energy substrate, and there are also reports that (RS)-3-sodium hydroxybutyrate administered intravenously to patients under a "very-low-energy diets" therapy proved to be effective in suppressing protein catabolism [(1) Rawan G.L.S., Semple S.L.G., "Lancet," 1, 15 - 17, 1983; (2) Sherwin RS, Hendler R.G., Felic P., "The Journal of Clinical Investigation," 55, 1382 - 1390). These are reports of the cases in which this substance was administered to the living body under the forced condition of scarcity of glucose. The present inventors, however, made rather extensive studies of traumatized patients or severely burned patients in the acute phase, post-operative patients with strong indications of surgical aggression, patients in hepatic insufficiency, patients unable to take food orally, patients in the state of metabolic (except diabetes-induced) acidosis and found and confirmed that many of such patients were falling into the state of abnormality of metabolism with regard to glucose, amino acid et cetera, this resulting in insufficient utilization of intravenously-administered nutrients such as sugar, amino acids and fat, and they were forced to use energy produced inside through decomposition of protein in their bodies and also that the levels in blood of alanine and glutamine resulting from decomposition of protein were increasing and due to the simultaneous loss of nitrogen, the cumulative nitrogen equilibrium in blood was markedly biased toward the minus side. Patients under such conditions are very likely to have their cells' immune function lowered and suffer from complication by serious infection diseases.

It is the object of the present invention to provide a substitution fluid preparation which patients can easily metabolize as an energy source and which is effective for suppressing increased protein catabolism in the living body.

This object could be solved by the surprising finding that substitution fluid preparations containing at least one of 3-hydroxybutyric acid having (R)-configuration and salts thereof can accomplish the aforementioned advantages.

The invention is described in detail in connection with the drawings in which

Fig. 1 shows the cumulative nitrogen equilibrium one week after injury in extensive cases of burn;

Fig. 2 is a graph showing the change-with-time of the concentrations in venous blood of glucose, amino acid and NEFA; and

Fig. 3 is another graph showing the change-with-time of the concentration in blood of alanine.

Fig. 4 is still another graph showing the change-with-time of the total amount of nitrogen contained in urine and the excreted amount of urea nitrogen.

The present invention relates to the provision of:

firstly, a substitution fluid preparation for the supply of energy to patients in increased protein catabolism in the living body which contains at least one of 3-hydroxybutyric acid having (R)-configuration and salts thereof in an amount effective to suppress said increased protein catabolism;

secondly, a substitution fluid preparation, wherein said substitution fluid preparation, which is intended for supplementing extracellular fluid by direct intraveneous administration, comprises 0.3 to 1.0 wt.% of 3-hydroxybutyric acid or inorganic salts thereof, pharmaceutically acceptable inorganic electrolytes and water, the pH of said preparation ranging from 4.5 to 8.0, and the osmotic pressure ratio ranging from 0.7 to 1.2;

thirdly, a substitution fluid preparation wherein said substitution fluid preparation, which is intended for maintaining good physical conditions by direct intravenous administration, comprises 0.18 to 3.0 wt.% of 3-hydroxybutyric acid or inorganic salts thereof, saccharides, pharmaceutically acceptable inorganic electrolytes and water, the pH of said preparation being from 4.0 to 8.0 and the osmotic pressure ratio being from 1.0 to 3.0;

fourthly, a substitution fluid preparation, wherein said substitution fluid preparation, which is intended for maintaining good physical conditions by direct intraveneous administration, comprises 100 to 8000 mg/ml of L-lysine salt of 3-hydroxybutyric acid, L-arginine salt of 3-hydroxybutyric acid and/or L-hystidine salt of 3-hydroxybutyric acid, amino acids and water, the pH of said preparation being from 5.0 to 8.0 and the osmotic pressure ratio being from 3 to 13;

fifthly, a substitution fluid preparation, wherein said substitution fluid preparation, which is intended for using to prepare substitution fluid preparation by direct intravenous administration, comprises 0.5 to 2.0 moles/1 of 3-hydroxybutyric acid, sodium 3-hydroxybutyric acid or potassium 3-hydroxybutiric acid and water, the pH being from 6.0 to 9.0;

sixthly, use of at least one of 3-hydroxybutyric acid having (R)-configuration and salts thereof for the preparation of a substitution fluid preparation for the supply of energy to patients in increased protein catabolism in the living body.

The patients in the state of increased protein catabolism in the living body include invasion-suffered patients by trauma, scald, postoperation, severe infection and the like, patients insufficient in hepatic functions and ketone-synthesizing ability and patients in malnutrition due to inability to take foods orally (protein-calorie malnutrition, in particular).

3-hydroxybutyric acid used in the present invention is preferably used as (R)-form or (RS)-form since 3-hydroxybutyric acid having (R)-configuration with respect to asymmetric carbon atom at 3-position is markedly effective as an energy source. As salts of 3-hydroxybutyric acid, inorganic salts such as sodium, and potassium are included, and further, salts of basic amino such as L-lysine, L-histidine and L-arginine are also usable. These can be used either alone or in combination. The salts of basic amino acids are effective, in particular, in the substitution fluid preparation of the present invention which is often usable for the purpose of supplying energy in conjunction with other nutritious agents such as amino acid-containing substitution fluid preparation.

The content of 3-hydroxybutyric acid or its salts is variable depending on the purpose or form of the substitution fluid preparation, but the lower limit is determined by the content which is effective in administering as an energy source to vein and the adjustment of osmotic pressure ratio, while the upper limit is optionally determined by osmotic pressure ratio, pH and the balance of nutrients.

Moreover, with respect to the content of 3-hydroxybutyric acid or its salts, the content of the (R)-form is different between the case where the (R)-form is used and the case where the (RS)-form is used, but each of them is usually used in the content as aforesaid from the adjustment of the osmotic pressure ratio. Accordingly, even when the (RS)-form is used at the lower content, it serves as supplying energy.

As inorganic electrolytes used as ingredient of substitution fluid for supplementing extracellular fluid and for maintaining good physical conditions are generally cited Ringer's substitution fluid preparation, glucose, maltose, sorbitol, mannitol, xylitol, alkali or alkali earth metal salts such as sodium chloride, potassium chloride and magnesium chloride which is added to various amino acid-fortified substitution fluid preparations; buffer salts such as sodium dihydrogen phosphate, potassium dihydrogen phosphate, sodium hydrogen phosphate and potassium hydrogen phosphate, and these, too, can be used either alone or in combination.

As sugars used in substitution fluid preparations are included, among others, reduced sugars such as grape sugar, maltose and fruit sugar; non-reduced sugars such as D sorbitol, mannitol and xylitol; polysaccharides such as dextran which is obtained by decomposition of starch, and these, too, can be used alone or in combination. As amino acids are included L-amino acid and its hydrochlorides, which, too, can be used either alone or in combination.

In preparing the substitution fluid of the present invention, each ingredient is first dissolved and the solution's pH and osmotic pressure ratio are adjusted. Especially when 3-hydroxybutyric acid is used, it itself has a high degree of acidity and pH adjustment by the use of sodium hydroxide is required.

For example, when addition to a substitution fluid for supplementing extracellular fluid or substitution fluid for maintaining good physical conditions is intended, hydroxybutyric acid or its salt or both of the foregoings are to be dissolved in water preferably to a concentration of 0.5 - 2 mol/liter and its pH is to be preferably be adjusted to a range of 6 - 8. They are usable with both concentration and pH outside of the aforementioned ranges but as to concentration, handling is easy when it is within the aforementioned range, and as to pH, too, it is advisable to have it controlled within the given range, for in that case possible correction of the pH can be precluded.

Concrete examples of substitution fluid compositions are given below.

(1) Substitution fluid composition intended for addition to substitution fluid for supplementing extracellular fluid or to substitution fluid for maintaining good physical conditions.

| Composition (a) | |
|---|---|
| 3-hydroxybutyric acid [(RS) or (R)-form] : | 5.2 - 10.4 wt.% |
| Sodium hydroxide : | Quantity required to adjust pH of substitution fluid within a range of 6.0 - 8.0 |
| Distilled water for injection : | Quantity required for dilution to the aforementioned concentration |
| Osmotic pressure ratio : | 3 - 6 |

| Composition (b) | |
|---|---|
| 3-sodium hydroxybutyrate [(RS) or (R)-form] : | 6.3 - 12.6 wt.% |
| Distilled water for injection : | Quantity required for dilution to the aforementioned concentration |
| pH : | 6.0 - 8.0 |
| Osmotic pressure ratio : | - 6 |

Composition (c)

3-hydroxybutyric acid ((RS) or (R)-form) :

10.4 - 20.8 wt.%

Sodium hydroxide : Quantity required to adjust pH of substitution fluid within a range of 6.0 - 9.0

Distilled water Quantity required for dilution to the for injection : aforementioned concentration

Osmotic pressure ratio : 3 - 11

| Composition (d) | |
|---|---|
| 3-sodium hydroxybutyrate [(RS) or (R)-form] : | 5.2 - 10.4 wt.% |
| Sodium hydroxide : | Quantity required to adjust pH of substitution fluid within a range of 8.0 - 9.0 |
| for injection : | aforementioned concentration |
| Osmotic pressure ratio : | 3 - 11 |

| Composition (e) | |
|---|---|
| 3-hydroxybutyric acid [(RS) or (R)-form] : | 5.2 - 10.4 wt.% |
| Sodium hydroxide : | Quantity required to adjust pH of substitution fluid within a range of 6.0 - 9.0 |
| Distilled water for injection : | Quantity required for dilution to the aforementioned concentration |
| Osmotic pressure ratio : | 6 - 11 |

Composition (f)

3-sodium hydroxybutyrate [(RS) or (R)-form] :

12.6 - 25.2 wt.%

Distilled water    Quantity required for dilution to the

for injection :    aforementioned concentration

pH            :    6.0 - 9.0

Osmotic pressure ratio : 3 - 11

| Composition (g) | |
|---|---|
| 3-hydroxybutyric acid [(RS) or (R)-form] : Distilled water for injection : pH : Osmotic pressure ratio : | 6.3 - 12.6 wt.% Quantity required for dilution to the aforementioned concentration 8.0 -9.0 3 - 11 |

| Composition (h) | |
|---|---|
| 3-sodium hydroxybutyrate [(RS) or (R)-form] : Distilled water for injection : pH : Osmotic pressure ratio : | 6.3 - 12.6 wt.% · Quantity required for dilution to the aforementioned concentration 6.0 - 8.0 3 - 11 |

(2) Substitution fluid preparation as supplementary liquid for extracellular fluid

| Composition (a): | |
|---|---|
| 3-sodium hydroxybutyrate | 0.35 - 1.0 wt.% |
| Sodium chloride | 0.41 - 0.6 wt.% |
| Potassium chloride | 0.03 wt.% |
| Calcium chloride | 0.02 wt.% |
| To be dissolved with distilled water for injection. | |
| pH : Osmotic pressure ratio : | 4.5 - 8.0 0.7 - 1.2 |

6

| Composition (b): | |
|---|---|
| 3-hydroxybutyric acid<br>Sodium hydroxide<br>Sodium chloride<br>Potassium chloride<br>Calcium chloride | 0.30 - 0.85 wt.%<br>Quantity required to adjust the solution pH to the preset level.<br>0.41 - 0.6 wt.%<br>0.03 wt.%<br>0.02 wt.% |
| To be dissolved with distilled water for injection. | |
| pH :<br>Osmotic pressure ratio : | 4.5 - 8.0<br>0.7 - 1.2 |

(3) Substitution fluid preparation as a basic fluid for maintenance substitution fluid

| Composition (a): | |
|---|---|
| 3-sodium hydroxybutyrate [(RS) or (R)-form]<br>Saccharide<br>Sodium chloride<br>Potassium chloride<br>pH :<br>Osmotic pressure ratio : | 0.2 - 0.6 wt.%<br>2.0 - 5.0 wt.%<br>0.09 wt.%<br>0.149 wt.%<br>4.0 - 8.0<br>1.0 - 1.6 |

| Composition (b): | |
|---|---|
| 3-hydroxybutyrate [(RS) or (R)-form]<br>Saccharide<br>Sodium chloride<br>Potassium chloride<br>Sodium hydroxide<br>pH :<br>Osmotic pressure ratio : | 0.18 - 0.5 wt.%<br>2.0 - 5.0 wt.%<br>0.09 wt.%<br>0.149 wt.%<br>The quantity required for adjustment to the preset pH<br>4.0 - 8.0<br>1.0 - 1.6 |

| Composition (c): | |
|---|---|
| 3-sodium hydroxybutyrate [(RS) or (R)-form]<br>Saccharide<br>Sodium chloride<br>Potassium chloride<br>pH :<br>Osmotic pressure ratio : | 0.6 - 3.0 wt.%<br>2.0 - 5.0 wt.%<br>0.09 wt.%<br>0.149 wt.%<br>4.0 - 8.0<br>1.0 - 3.0 |

| Composition (d): | |
|---|---|
| 3-hydroxybutyrate [(RS) or (R)-form] | 0.2 - 0.6 wt.% |
| Saccharide | 2.0 - 5.0 wt.% |
| Sodium chloride | 0.09 wt.% |
| Potassium chloride | 0.149 wt.% |
| pH : | 4.0 - 8.0 |
| Osmotic pressure ratio : | 1.0 - 3.0 |

| Composition (e): | |
|---|---|
| 3-sodium hydroxybutyrate [(RS) or (R)-form] | 0.5 - 2.4 wt.% |
| Saccharide | 2.0 - 5.0 wt.% |
| Sodium chloride | 0.09 wt.% |
| Potassium chloride | 0.149 wt.% |
| Sodium hydroxide | The quantity required for adjustment to the preset pH |
| pH : | 4.0 - 8.0 |
| Osmotic pressure ratio : | 1.0 - 3.0 |

| Composition (f): | |
|---|---|
| 3-hydroxybutyrate [(RS) or (R)-form] | 0.18 - 2.4 wt.% |
| Saccharide | 2.0 - 5.0 wt.% |
| Sodium chloride | 0.09 wt.% |
| Potassium chloride | 0.149 wt.% |
| Sodium hydroxide | The quantity required for adjustment to the preset pH |
| pH : | 4.0 - 8.0 |
| Osmotic pressure ratio : | 1.6 - 3.0 |

The aforementioned compositions for the individual substitution fluid preparations are given only as examples and are not to be taken as limiting.

(4) Substitution fluid preparation for amino acid containing substitution fluid preparation

8

|  | mg (per 100 ml of substitution fluid) |
|---|---|
| L-isoleucine | 500 ~ 950 |
| L-leucine | 400 ~ 1500 |
| L-methyonine | 200 ~ 1200 |
| L-phenylalanine | 100 ~ 1300 |
| L-threonine | 100 ~ 700 |
| L-tryptophan | 50 ~ 300 |
| L-valine | 200 ~ 900 |
| L-cystine | 10 ~ 150 |
| L-tyrosine | 30 ~ 70 |
| L-histidine | 240 ~ 800 |
| L-alanine | 450 ~ 900 |
| L-aspartic acid | 50 ~ 400 |
| L-glutamic acid | 50 ~ 700 |
| L-proline | 200 ~ 1100 |
| L-serine | 200 ~ 500 |
| Aminoacetate | 500 ~ 1900 |
| L-lysin (3-hydroxybutyrate) | 100 ~ 8000 |
| L-arginine (3-hydroxybutyrate) | 100 ~ 8000 |

Each of amino acids may be optionally omitted depending on the purpose of the substitution fluid.

The substitution fluid composition so prepared is filled in vials or like containers made of a plastic inert to the preparation and then, with the mouth or inlet sealed, sterilized with steam together with the container. Administration of the aforementioned substitution fluid preparation to patients is done in one of the ways exemplified below.

Case 1: 80 - 300 ml/day of the preparation is first diluted to not less than "ana" (equivalent) using distilled water for injection or an existing substitution fluid for supplementing extracellular fluid or for maintaining good physical conditions, and then administered at a rate not exceeding 100 milli equivalent/hour as 3-hydroxybutyric acid by intravenous drip injection.

Case 2: 500 - 1,000 ml per time is administered at a rate of 50 - 1,000 ml/hour by intravenous drip injection.

Case 3: 500 - 1,000 ml per time is administered as grape sugar at a rate of not more than 0.5 g/hour/kg (body weight) by intravenous drip injection. From the viewpoint of calorie, it is preferred to adjust the amount used to 2000 - 2800 Cal. for high calorie administration from central viein and to approximately 1000 Cal. for the administration from peripheral vein.

Needless to say, however, the dose and administration rate for any such substitution fluid preparation are to be adjusted properly with the condition of the patient taken into due consideration.

The substitution fluid preparation of the present invention is often supplied to patients who obtain energy by decomposition of protein in the living body because of insufficient uptake of saccharides such as glucose, and hence recovery of the patients can be accelerated more effectively by the conjoint use with other nutritious agents like amino acids and the like or by the conjoint use with saccharides.

Hereinafter, the present invention is described in greater detail, giving examples, but, needless to say, this invention is in no way limited thereby.

Example 1

1 mol of (RS)-3-hydroxybutyric acid was dissolved in 100 ml of distilled water for injection and then neutralized with sodium hydroxide solution added dropwise to pH > 8.0 and distilled water was further added to make up the whole to 1 liter. This was filled in a 1-liter plastic container and, with the mouth of the container sealed, sterilized by heat treatment for 2 hours at 110°C. The resulting liquid composition was 8.0 in pH and 6 in osmotic pressure ratio. Then distilled water for injection was added to the aforementioned 250 ml of liquid composition to make up the whole to 1 liter, again filled in the plastic container and sterilized in a like manner to prepare the substitution fluid preparation.

9

Example 2

A substitution fluid preparation containing (RS)-3-sodium hydroxybutyrate and inorganic hydrolytes which is suited in composition for use as a substitution fluid for supplementing extracellular fluid was prepared in the following way.

| (RS)-3-sodium hydroxybutyrate | 9.70 g |
|---|---|
| Sodium chloride | 4.1 g |
| Potassium chloride | 3.0 g |
| Calcium chloride | 2.0 g |

The above ingredients were dissolved with distilled water for injection to make up the whole to 1 liter. The resulting liquid composition was 7.2 in pH and 1 in osmotic pressure ratio. The electrolyte composition was as follows

$Na^+$     154 milli equivalent/liter

$K^+$     4    〃

$Ca^{++}$     3    〃

$Cl^-$     77    〃

$CH_3CHCH_2COO^-$     77    〃
     |
     OH

The above liquid composition was filled in a 1-liter plastic container and then, the mouth of the container was sealed, sterilized in the same way as described in Example 1.

Example 3

A substitution fluid for maintaining good physical conditions containing (RS)-3-sodium hydroxybutyrate, grape sugar and inorganic electrolytes was prepared as follows.

| Grape sugar | 43 g |
|---|---|
| (RS)-3-sodium hydroxybutyrate | 4.41 g |
| Sodium chloride | 0.9 g |
| Potassium chloride | 1.49 g |
| Sodium hydroxide | Quantity needed to adjust pH to 7.0. |

The above ingredients were dissolved and diluted with distilled water for injection to make up the whole to 1 liter. The resulting liquid composition was 7.0 in pH and 1.3 in osmotic pressure ratio. The electrolyte composition was as follows.

| | |
|---|---|
| Na⁺ | 50 milli equivalent/liter |
| K⁺ | 20 〃 |
| Cl⁻ | 35 〃 |
| $CH_3CHCH_2COO^-$<br>　　|<br>　　OH | 35 〃 |

Example 4

A substitution fluid preparation containing (R)-3-hydroxybutyric acid and inorganic electrolytes, whose composition is similar to that of a substitution fluid for supplementing extracellular fluid was prepared in the following way.

| | |
|---|---|
| (R)-3-hydroxybutyric acid | 3.5 g |
| Sodium chloride | 6.0 g |
| Potassium chloride | 3.0 g |
| Calcium chloride | 2.0 g |
| Sodium hydroxide | Quantity needed to adjust pH to 7.0. |

The above ingredients were dissolved and diluted with distilled water for injection to make up the whole to 1 liter. The resulting liquid composition was 1 in osmotic pressure ratio. The electrolyte composition was as follows.

| | |
|---|---|
| Na⁺ | 130 milli equivalent/liter |
| K⁺ | 4 〃 |
| Ca⁺⁺ | 3 〃 |
| Cl⁻ | 109 〃 |
| $CH_3CHCH_2COO^-$<br>　　|<br>　　OH | 28 〃 |

The above liquid composition was filled in a plastic container and, with the mouth of the container sealed, sterilized in the same way as described in Example 1.

Example 5

A high-calorie maintaining type of substitution fluid preparation containing (R)-3-potassium hydroxybutyrate and (R)-3-sodium hydroxybutyrate was prepared in the following way. The composition of the substitution fluid preparation for maintenance (700 ml) was as follows.

| Grape sugar | 175 g |
|---|---|
| (R)-3-potassium hydroxybutyrate | 3.1 g |
| (R)-3-sodium hydroxybutyrate | 1.8 g |
| Magnesium sulfate (heptahydrate) | 1.24 g |
| Potassium dihydrogen phosphate | 0.66 g |
| Zinc sulfate (heptahydrate) | 0.003g |
| Potassium gluconate | 1.19 g |

The electrolyte composition of the above substitution fluid preparation was as follows.

$$Na^{\cdot} \qquad\qquad 20 \text{ milli equivalent/liter}$$

$$K^{\cdot} \qquad\qquad 30 \qquad\qquad ''$$

$$Mg^{\cdot} \qquad\qquad 10 \qquad\qquad ''$$

$$CH_3\underset{\underset{OH}{|}}{C}HCH_2COO^{-} \qquad 41.5 \qquad\qquad ''$$

Example 6

A substitution fluid containing L-lysine (3-hydroxybutyrate) and L-arginine (3-hydroxybutyrate) was prepared according to the manner as mentioned below;

That is, 800 mg of L-isoleucine, 1400 mg of L-leucine, 1500 mg of L-lysine (3-hydroxybutyrate), 400 mg of L-methyonine, 500 mg of L-phenylalanine, 400 mg of L-threonine, 200 mg of L-tryptophan, 800 mg of L-valine, 100 mg of L-cystine, 50 mg of L-tyrosine, 2000 mg of L-arginine (3-hydroxybutyrate), 300 mg of L-histidine, 450 mg of L-alanine, 100 mg of L-aspartic acid, 400 mg of L-glutamic acid, 300 mg of L-proline, 300 mg of L-serine were dissolved into 100 ml of distilled water and sterilized.

The pH and the osmotic pressure ratio were as shown below;

pH : 7.0

Osmotic pressure ratio : 4

Application Example 1

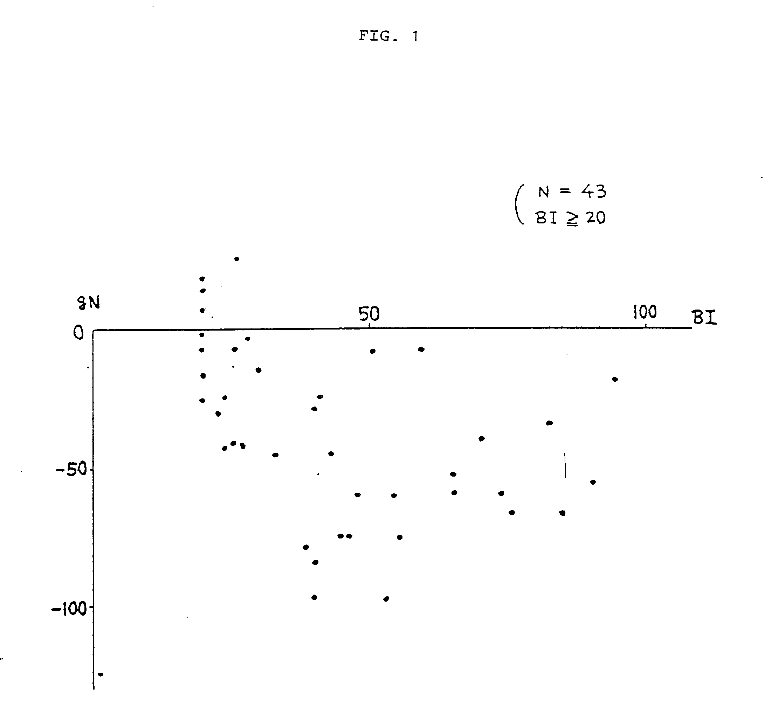

The substitution fluid preparation of Example 1 was administered to a male severely burned patient in the acute phase (the burn had its center on the front side of the chest and of the total injured area of 33 %, 13 % was in the phase III) into the central vein for 3 hours at a rate of 25 micro-mol/Kg/min. The result is shown in Figs. 1 through 3.

Fig. 1 shows the progress of the cumulative nitrogen equilibrium in one week after the injury for patients not less than 20 in burn index, and from Fig. 1 it is apparent that the loss of nitrogen in the early phase of injury due to increased protein catabolism is unignorably large. It is known that alanine as a glycogenic amino acid flows out through the peripheral tissues, which was caused by protein catabolism.

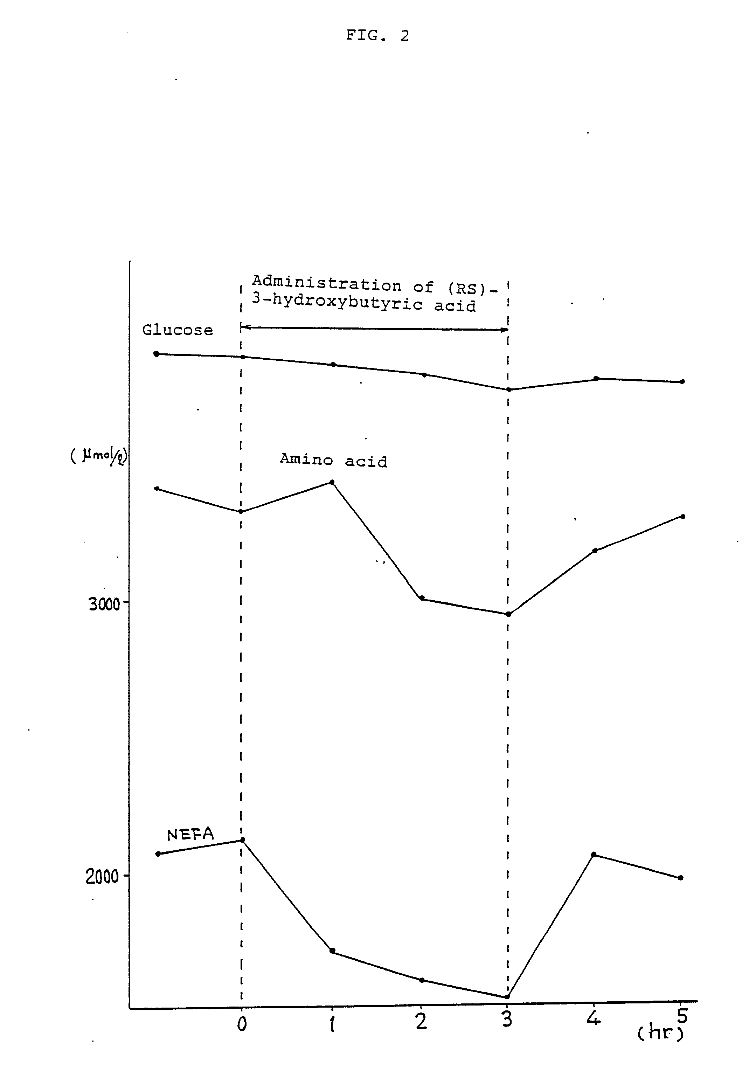

Fig. 2 shows that the concentrations in blood of the total amino acid and free fatty acid lower significantly as administration of (RS)-3-hydroxybutyric acid is continued.

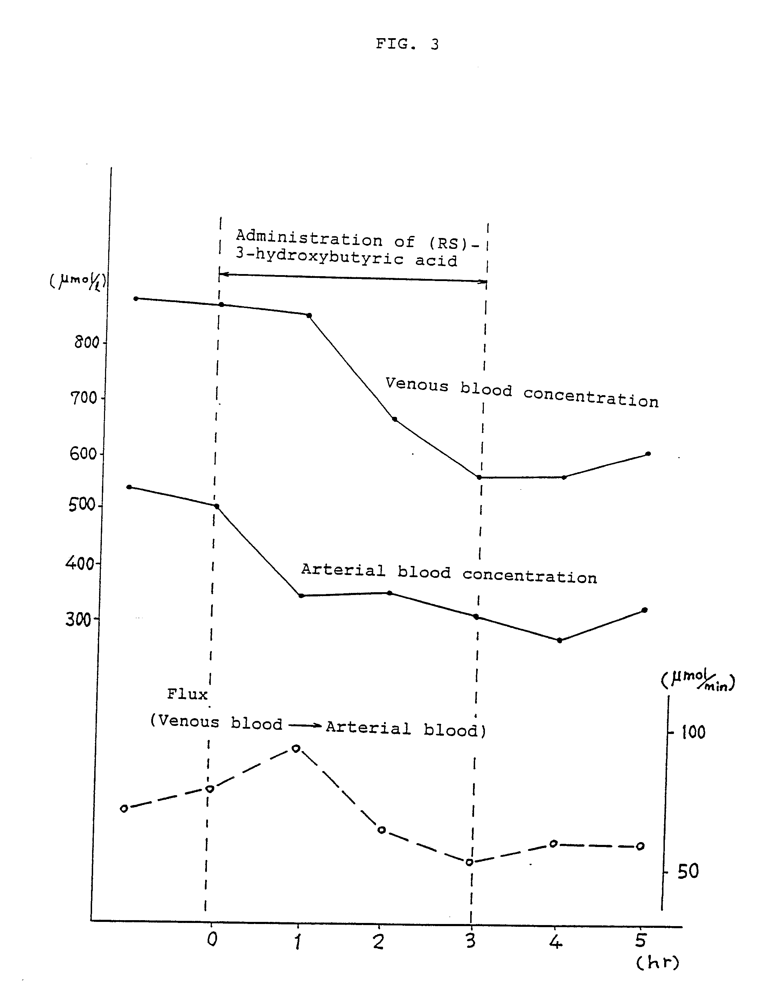

Fig. 3 shows the concentration in blood of alanine and flux before and after administration of (RS)-3-hydroxybutyric acid, and from Fig.3 it is apparent that the alanine concentration is markedly dropped as its administration is continued. The fact that of the amino acids present in blood, alanine closely related with protein catabolism has its concentration decreased significantly suggests that (RS)-3-hydroxybutyric acid administered when the butn is in the acute phase in which the patient is difficult to take nutrients is effective for saving of the own protein.

12

Application Example 2

Study was made, by use of 4 severely traumatized patients, to see what kind of changes with regard to metabolism is caused by addition of (RS)-3-sodium hydroxybutyrate to a substitution preparation including glucose for maintaining good physical conditions.

In a test with 700 ml of Hicalic #2® (Termo) and 200 ml of Proteamin 12 ® (Tanabe Seiyaku) as the basic substitution fluid preparation, the solution described in Example 1 above prepared by dissolving (RS)-3-sodium hydroxybutyrate in distilled water for injection was added thereto, and then the mixed preparation was administered for 3 hours at a rate equivalent of 25 micromol/kg/min. of (RS)-3-sodium hydroxybutyrate and the change-with-time of the concentration of 3-hydroxybutyric acid, difference thereof between femoral artery and vein, blood sugar and alanine concentration were measured. The administration was made 3 days after injury. The results are shown in Table 1.

Table 1    (Unit: micromol/liter)

| | Change-with-time of concentration in blood | | | | |
|---|---|---|---|---|---|
| | 0 | 2hrs | 3hrs | 4hrs | 5hrs |
| Concentration of 3-hydroxybutyric acid  (Artery) | 213 ± 73 | 1382 ± 302 | 1432 ± 412 | 438 ± 121 | 313 ± 97 |
| Difference between femoral artery and vein | 44 ± 50 | 492 ± 263 | 432 ± 203 | 213 ± 112 | 23 ± 61 |
| Concentration of blood sugar (Vein) | 7683 ± 1232 | 7243 ± 1252 | 7462 ± 1513 | 7513 ± 1216 | 7522 ± 1317 |
| Concentration of alanine (Vein) | 332 ± 101 | 291 ± 111 | 273 ± 163 | 283 ± 121 | 300 ± 119 |
| Administration of 3-sodium hydroxy-butyrate | (25 micromol/Kg/min.) | | | | |

From the test results shown in Table 1, it was recognized that the concentration in blood of (RS)-3-sodium hydroxybutyrate is increased even when it is added simultaneously with glucose, that the difference between femoral artery and vein is increased, this indicating that the administered (RS)-3-sodium hydroxybutyrate is consumed in the peripheral tissues of the lower limbs and that the concentration in blood of alanine is significantly lowered by administration of (RS)-3-sodium hydroxybutyrate. This, again, proves its effect to save the own protein in the living body.

Application Example 3

A test was made to see if (R)-3-sodium hydroxybutyrate is more effective than (RS)-3-sodium hydroxybutyrate.

The test was carried out using hemorrhagic shock rats to compare the effects of administration of substitution fluid preparations for supplementing extracellular fluid containing (R)-3-sodium hydroxybutyrate and (RS)-3-sodium hydroxybutyrate respectively with those of Ringer's solution containing lactic acid as control.

(1) Preparation of hemorrhagic shock rat model

13

With the carotid artery cannulated, the blood pressure was kept at 40 mmHg by the reserved bottle method.

The blood-removing time was set at 10 min. The shock continuing time was set at 30 min. and 1 ml of blood was taken every 10 min.

(2) Preparation of substitution fluid preparations for supplementing extracellular fluid containing (R)-3-sodium hydroxybutyrate and (RS)-3-sodium hydroxybutyrate

As electrolytes 0.41 wt.% of sodium chloride, 0.03 wt. % of potassium chloride and 0.02 wt.% of calcium chloride were used and the substitution fluid compositions were prepared with the contents of (R) and (RS)-3-sodium hydroxybutyrate adjusted to 0.96 wt.% respectively.

As control, a substitution fluid preparation containing sodium lactate of the following composition was prepared: Sodium chloride 0.41 wt.%, potassium chloride 0.03 wt.%, calcium chloride 0.02 wt.% and sodium lactate 0.863 wt.%

Administration of these substitution fluid preparations for supplementing extracellular fluid into rats was started immediately after the start of blood-removal, and it was continued for 30 min. at a rate of 15 micromol/kg/min.

The concentrations of total ketones in the blood samples taken up till 30 min. are shown in Table 2, and those of alanine in Table 3.

Table 2

| (Unit: micromol/liter) | | | | |
|---|---|---|---|---|
| | 0 | 10 min. | 20 min. | 30 min. |
| (R)-3-sodium-hydroxybutyrate group | 483 ± 218 | 1846 ± 257 | 1762 ± 379 | 1850 ± 309 |
| (RS)-3-sodium-hydroxybutyrate group | 435 ± 118 | 989 ± 380 | 931 ± 181 | 916 ± 201 |
| Sodium lactate group | 432 ± 117 | 293 ± 74 | 237 ± 49 | 228 ± 44 |

Table 3

| (Unit: micromol/liter) | | | | |
|---|---|---|---|---|
| | 0 | 10 min. | 20 min. | 30 min. |
| (R)-3-sodium-hydroxybutyrate group | 458 ± 100 | 478 ± 107 | 491 ± 75 | 491 ± 80 |
| (RS)-3-sodium-hydroxybutyrate group | 498 ± 126 | 559 ± 70 | 612 ± 80 | 710 ± 117 |
| Sodium lactate group butyrate group | 438 ± 154 | 552 ± 146 | 666 ± 136 | 782 ± 195 |

The data in Table 2 and Table 3 above show the following facts.

i) Compared with the sodium lactate group, the (R) and (RS) groups have the concentration in blood of total ketones increased, this value being significantly higher with the (R) group than with the (RS) group.

ii) Compared with the sodium lactate group, the (R) and (RS) groups are found having the rise of concentration better controlled as the shock progresses, this controlling effect being more significant with the (R) group than with the (RS) group.

iii)The (R) group has the alanine concentration-in-blood significantly better controlled than the (RS) group, having thus the own protein better saved as the shock progresses.

Application Example 4

14

In nutrient control of a 63-aged female patient of pneumonia, test was conducted by the use of a high calorie basic substitution fluid (A fluid) prepared by Example 5 and an amino acid-containing substitution fluid (B fluid).

As control, a commercially available Hicalic #2® (Termo) as high caloric basic substitution fluid and a commercially available Amiparen ® (Otsuka pharmaceutical) as amino acid-containing substitution fluid were used. The administration was made according to protocol shown by Table 4. During this period, the administration of an antibiotic agent was not changed.

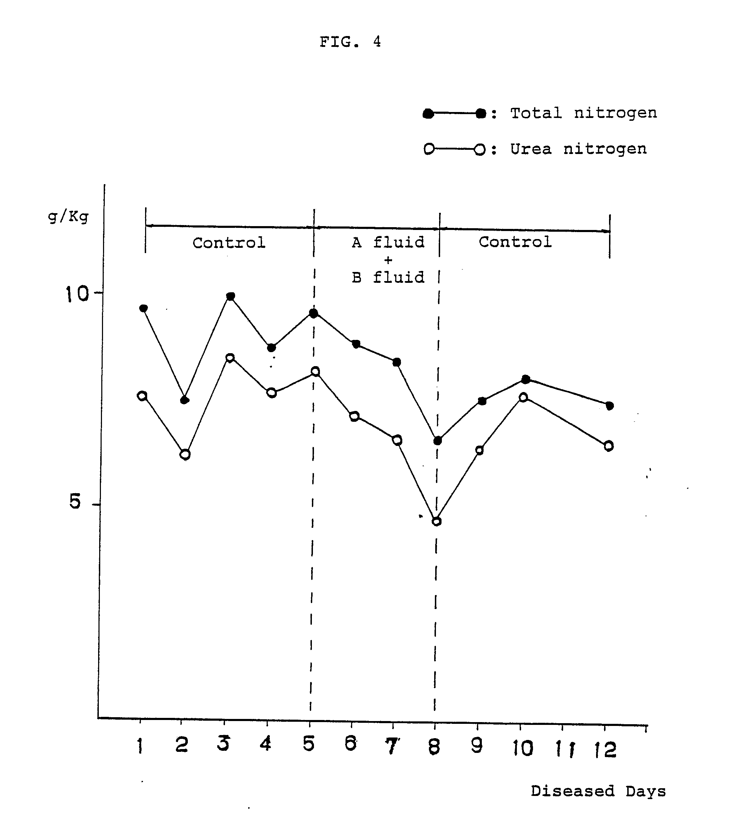

As a result of administration, the excreted amount of nitrogen in urine of the patient, as shown by Fig. 4, markedly decreased in 3 days during which both fluids A and B were used, which exhibited an effect of suppressing increased protein catabolism caused by systemic infection. Thereafter, the excreted amount of nitrogen in urine decreases to thus presumably contribute to remission of systemic conditions of the patient.

Table 4

| Diseased days | 1 | 2 | 3 | 4 | 5 | 6 | 7 | 8 | 9 | 10 | 11 | 12 |
|---|---|---|---|---|---|---|---|---|---|---|---|---|
| Administered preparation | Control | | | | | A fluid + B fluid | | | Control | | | |
| Administration (ml/day) | Hicalic #2® 2100 Amiparen® 600 | | | | | A fluid 2100 B fluid 600 | | | Hicalic #2® 2100 Amiparen® 600 | | | |
| Amount of heat administered (Kcal) | 2150 | | | | | 2230 | | | 2150 | | | |
| Amount of nitrogen administered (g/day) | 12.56 | | | | | 12.56 | | | 12.56 | | | |

As described above, 3-hydroxybutyric acid or its salts shows a marked effect for suppressing increased protein catabolism. Administration of the substitution fluid preparation of the present invention to patients such as those mentioned above, therefore, is highly effective in helping maintain their physical stamina and improve their immune function.

The substitution fluid preparation of the present invention is of quite a new type not having been studied heretofore in the field, being quite valuable from a physiological as well as nutrient viewpoint.

Claims

1. A substitution fluid preparation for the supply of energy to patients in increased protein catabolism in the living body which contains at least one of (R)-form of 3-hydroxybutyric acid and salts thereof in an amount effective to suppress said increased protein catabolism.

2. A substitution fluid preparation in accordance with Claim 1, wherein said salt of 3-hydroxybutyric acid is 3-sodium hydroxybutyrate, 3-potassium hydroxybutyrate, L-lysine salt of 3-hydroxybutyric acid, L-histidine salt of 3-hydroxybutyric acid or L-arginine salt of 3-hydroxybutyric acid.

3. A substitution fluid preparation in accordance with Claim 1 or 2, wherein said (R)-form of 3-hydroxybutyic acid and salts thereof is given as (RS)-hydroxybutyric acid and salts thereof or (R)-3-hydroxybutyric acid and salts thereof.

4. A substitution fluid preparation in accordance with any one of Claims 1 through 3, wherein said substitution fluid preparation, which is intended for supplementing extracellular fluid by direct intraveneous administration, comprises 0.3 to 1.0 wt.% of 3-hydroxybutyric acid or inorganic salts thereof, pharmaceutically acceptable inorganic electrolytes and water, the pH of said preparation being from 4.5 to 8.0, and the osmotic pressure ratio being from 0.7 to 1.2.

5. A substitution fluid preparation in accordance with any one of Claims 1 through 3, wherein said substitution fluid preparation, which is intended for maintaining good physical conditions by direct intravenous administration, comprises 0.18 to 3.0 wt.% of 3-hydroxybutyric acid or inorganic salts thereof, saccharides, pharmaceutically acceptable inorganic electrolytes and water, the pH of said preparation being from 4.0 to 8.0 and the osmotic pressure ratio being from 1.0 to 3.0.

6. A substitution fluid preparation in accordance with any one of Claims 1 through 3, wherein said substitution fluid preparation, which is intended for maintaining good physical conditions by direct intraveneous administration, comprises 100 to 8000 mg/ml of L-lysine salt of 3-hydroxybutyric acid, L-arginine salt of 3-hydroxybutyric acid and/or L-hystidine salt of 3-hydroxybutyric acid, amino acids and water, the pH of said preparation being from 5.0 to 8.0 and the osmotic pressure ratio being from 3 to 13.

7. A substitution fluid preparation in accordance with Claim 6, wherein said substitution fluid preparation contains saccharides and/or physiologically acceptable inorganic electrolytes.

8. A substitution fluid preparation in accordance with any one of Claims 1 through 3, wherein said substitution fluid preparation, which is intended for using to prepare substitution fluid preparation by direct intravenous administration, comprises 0.5 to 2.0 moles/1 of 3-hydroxybutyric acid, sodium 3-hydroxybutyric acid or potassium 3-hydroxybutiric acid and water, the pH beign from 6.0 to 9.0.

9. A substitution fluid preparation in accordance with Claim 4, 5 or 7, wherein said electrolyte is at least one alkali or alkali earth metal salt, e.g. sodium chloride, potassium chloride and magnesium chloride, and/or buffer salt, e.g sodium hydrogen phosphate, potassium hydrogen phosphate, sodium dihydrogen phosphate or potassium dihydrogen phosphate.

10. A substitution fluid preparation in accordance with Claim 5 or 7, wherein said saccharide is at least one reduced sugar, e. g. grape sugar, maltose or fruit sugar, non-reduced sugar, e. g. D-sorbitol, mannitol or xylitol, or polysaccharide which is obtained through decomposition of starch such as dextran.

11. A substitution fluid preparation in accordance with Claim 6, wherein said amino acid is an L-amino acid.

12. A substitution fluid preparation in accordance with any one of Claims 1 through 7, wherein said patients in increased protein catabolism in the living body is a invasion-suffered patient, patient insufficient in hepatic functions, patient unable to take food orally or a patient in the state of malnutrition.

13. Use of at least one of 3-hydroxybutyric acid having (R)-configuration and salts thereof for the preparation of a substitution fluid preparation for the supply of energy to patients in increased protein catabolism in the living body.

14. Use of at least one of 3-hydroxybutyric acid having (R)-configuration and salts thereof for the preparation of the substitution fluid preparation in accordance with any one of Claims 4 through 8.

FIG. 1

$\left( \begin{array}{l} N = 43 \\ BI \geqq 20 \end{array} \right.$

FIG. 2

FIG. 3

Administration of (RS)-3-hydroxybutyric acid

Venous blood concentration

Arterial blood concentration

Flux
(Venous blood → Arterial blood)

($\mu$mol/l)

($\mu$mol/min)

40

FIG. 4

●———●: Total nitrogen

○———○: Urea nitrogen

Diseased Days